(19) Europäisches Patentamt — European Patent Office — Office européen des brevets

(11) **EP 4 454 690 B1**

(12) **EUROPÄISCHE PATENTSCHRIFT**

(45) Veröffentlichungstag und Bekanntmachung des Hinweises auf die Patenterteilung:
**01.07.2026 Patentblatt 2026/27**

(51) Internationale Patentklassifikation (IPC):
***A61M 16/00*** *(2006.01)*

(52) Gemeinsame Patentklassifikation (CPC):
**A61M 16/0066;** A61M 16/107; A61M 2205/42

(21) Anmeldenummer: **24168371.3**

(22) Anmeldetag: **04.04.2024**

(54) **BEATMUNGSGERÄT**

RESPIRATORY APPARATUS

APPAREIL RESPIRATOIRE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **25.04.2023 DE 102023110582**

(43) Veröffentlichungstag der Anmeldung:
**30.10.2024 Patentblatt 2024/44**

(73) Patentinhaber: **Löwenstein Medical Technology S.A.**
**2557 Luxembourg (LU)**

(72) Erfinder: **Gerlach, Angela**
**22549 Hamburg (DE)**

(74) Vertreter: **Löwenstein Medical IP**
**Löwenstein Medical Technology**
**GmbH + Co.KG IP Management**
**Kronsaalsweg 40**
**22525 Hamburg (DE)**

(56) Entgegenhaltungen:
**US-A1- 2004 035 422    US-A1- 2014 299 132**
**US-A1- 2015 023 782    US-A1- 2016 015 919**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Beatmungsgerät. Ein Beatmungsgerät ist eine Maschine zur Beatmung von Personen mit unzureichender oder ausgesetzter Eigenatmung. Beatmungsgeräte umfassen in der Regel einen Einlass, einen Auslass sowie ein Gebläse. Das Gebläse dient dazu, ein Atemgas zu befördern. Unter einem Atemgas soll im Rahmen der vorliegenden Offenbarung ein Gas verstanden werden, das zu Beatmungszwecken von einem Beatmungsgerät befördert wird. Zwischen dem Einlass und dem Auslass bildet das Beatmungsgerät einen Atemgaspfad, in dem das Gebläse angeordnet sein kann. Der Atemgaspfad kann beispielsweise durch Gänge, Kanäle, Kammern und dergleichen gebildet werden, aber auch durch funktionale Komponenten des Beatmungsgeräts, wie etwa das Gebläse, hindurch geführt sein.

Bei Beatmungsgeräten ist die Schallunterdrückung eine bedeutende Herausforderung. Der bei der Beatmung notwendige Druck erfordert Gebläse, die mit einer hohen Drehzahl arbeiten. Diese Gebläse können regelmäßig Drehzahlen von bis zu 60.000 U/min oder sogar darüber hinaus leisten. Von Gebläsen geht bei entsprechend hohen Drehzahlen jedoch auch eine große Schallabstrahlung aus. Es ergibt sich ein relativ hoher Geräuschpegel, den man zu vermindern versucht. Zur Verminderung der Betriebsgeräusche wird nach dem Stand der Technik regelmäßig Schaum in Kombination mit einer Schalldämmstruktur eingesetzt.

So offenbaren beispielsweise die EP 1457222 B1 und die US 2016/015919 A1 Verfahren zur Schalldämmung bei der Beatmung, bei dem mindestens ein Teil eines Atemgases durch eine Dämpfungsbox hindurchgeleitet wird. Die Dämpfungsbox weist einen Strömungskanal auf, in dessen Bereich die Atemluft an einem herausnehmbaren schalldämmenden Material vorbeigeführt wird. Das schalldämmende Material ist vorliegend ein Schaumstoff oder ein Flies. Durch den verwendeten Schaumstoff ist ein erhöhter Materialaufwand notwendig und es werden zusätzliche Komponenten im Beatmungsgerät eingesetzt. Der Materialaufwand und somit die Kosten für das Beatmungsgerät sind erhöht. Durch die im Stand der Technik vorgeschlagene Verwendung eines schalldämmenden Materials erhöht sich zudem die Größe des Beatmungsgeräts, was jedoch sowohl aus Transportgründen als auch für den Platzbedarf im Einsatz als nachteilig angesehen wird. Darüber hinaus gestaltet sich auch die Herstellung des Beatmungsgeräts umständlicher, insbesondere durch den aufgrund der zusätzlichen Schaumstoff-Komponenten bedingten Mehraufwand bei der Montage. US 2014/299132 A1 offenbart ein CPAP-Gerät, das in einem Schlauch montiert und ausgebildet ist. US 2004/035422 A1 zeigt ein Beatmungsgerät mit zwei Leitungen und einer Ventilanordnung zur Geräuschreduzierung. US 2015/023782 A1 zeigt einen Flowgenerator mit einem Gebläse, das über eine druckabdichtende, elastomere Aufhängungsvorrichtung innerhalb des Gehäuses aufgehängt ist.

Der Erfindung liegt deshalb die Aufgabe zugrunde, ein Beatmungsgerät bereitzustellen, das weniger komplex hinsichtlich der Verwendung unterschiedlicher Materialien bzw. Komponenten bei gleichzeitig zufriedenstellender Reduktion der Betriebsgeräusche des Beatmungsgeräts ist. Es ist eine weitere Aufgabe der Erfindung, ein Beatmungsgerät mit Schallunterdrückung bereitzustellen, das kompakt und einfach in der Herstellung ist.

Die Aufgabe wird gelöst durch die Bereitstellung des Beatmungsgeräts nach Patentanspruch 1. In den abhängigen Patentansprüchen sind optionale Ausführungsformen gemäß vorliegender Offenbarung beschrieben, deren Merkmale im Rahmen des technisch sinnvollen beliebig miteinander kombiniert werden können.

Erfindungsgemäß wird ein Beatmungsgerät bereitgestellt, mit einem Einlass, mit einem Auslass und mit einem Gebläse, wobei zwischen dem Einlass und dem Auslass ein Atemgaspfad gebildet ist, der durch das Gebläse geführt ist. Entlang des Atemgaspfads ist eine Kammer, ein in die Kammer mündender erster Kanal und ein in die Kammer mündender zweiter Kanal angeordnet. Dabei weicht eine erste akustische Impedanz des ersten Kanals und eine zweite akustische Impedanz des zweiten Kanals von einer dritten akustischen Impedanz der Kammer ab, sodass ein durch das Gebläse erzeugter Schall gedämpft wird.

Die erste, zweite und dritte akustische Impedanz können jeweils als eine akustische Flussimpedanz verstanden werden. Die akustische Flussimpedanz ist der Widerstand, der einer Schallausbreitung, zum Beispiel in Rohren, entgegengesetzt wird. Bei einem Übergang zwischen Bereichen mit unterschiedlicher akustischer Impedanz kommt es zu Reflexionen, wodurch sich eine schalldämpfende Wirkung einstellt. Wie beobachtet wurde, führt die Bauart des Beatmungsgeräts gemäß vorliegender Offenbarung mit der Kammer sowie dem ersten Kanal und dem zweiten Kanal, die in die Kammer münden, zu einer Verringerung der von dem Beatmungsgerät ausgehenden Betriebsgeräusche. Dies ist dadurch bedingt, dass die dritte akustische Impedanz der Kammer von der ersten akustischen Impedanz des ersten Kanals und von der zweiten akustischen Impedanz des zweiten Kanals abweicht. Die erste akustische Impedanz des ersten Kanals kann, muss aber nicht zwangsläufig, mit der zweiten akustischen Impedanz des zweiten Kanals übereinstimmen. Mit anderen Worten besteht eine Impedanzfehlanpassung zwischen dem ersten Kanal und der Kammer sowie zwischen dem zweiten Kanal und der Kammer.

Um einen Unterschied der akustischen Impedanz zwischen der Kammer und dem ersten beziehungsweise dem zweiten Kanal zu erreichen, können die Kammer und der erste bzw. der zweite Kanal gemäß vorliegender Offenbarung unterschiedlich dimensioniert sein.

Gemäß einer Ausführungsform der vorliegenden Offenbarung weisen die Kammer und der erste bzw. der zweite Kanal einen unterschiedlichen Querschnitt und/oder ein unterschiedliches Volumen auf. Es ist vorteilhaft, wenn sich der Atemgaspfad an einem Übergang von dem ersten Kanal bzw. dem zweiten Kanal in die Kammer verbreitert bzw. aufweitet. Insbesondere kann ein Querschnitt des Atemgaspfads in dem ersten Kanal bzw. dem zweiten Kanal kleiner sein als innerhalb der Kammer. Gemäß einer anderen Ausführungsform der vorliegenden Offenbarung ist es hingegen vorgesehen, dass der Querschnitt des Atemgaspfads in dem ersten Kanal bzw. in dem zweiten Kanal größer ist als innerhalb der Kammer.

Insbesondere kann der Atemgaspfad, vorzugsweise der erste Kanal und/oder der zweite Kanal und/oder die Kammer, weiter vorzugsweise die Komponenten des Beatmungsgeräts entlang des Atemgaspfades, Schaumstoff-frei und/oder Fliesfrei ausgebildet sein.

Das Beatmungsgerät weist bevorzugt ein Gehäuse auf. Der Einlass und/oder der Auslass werden bevorzugt durch das Gehäuse gebildet. Vorzugsweise ist das Gebläse innerhalb des Gehäuses angeordnet. Der Atemgaspfad verläuft vorzugsweise innerhalb des Gehäuses. Hierzu können innerhalb des Gehäuses beispielsweise mehrere Kanäle und/oder Kammern angeordnet sein. Das Gehäuse, Außen- sowie Innenwände des Beatmungsgeräts sowie sonstige Bauteile des Beatmungsgeräts können gemäß vorliegender Offenbarung durch diverse Materialien gebildet sein, beispielsweise durch Metall und/oder Kunststoff.

Bei dem Gebläse des Beatmungsgeräts handelt es sich um eine Vorrichtung, die ein gasförmiges Medium befördern kann. Es kann sich dabei prinzipiell um einen Ventilator, einen Lüfter oder ein sonstiges Gebläse handeln. Gemäß einer bevorzugten Variante der vorliegenden Offenbarung ist das Gebläse ein Radialgebläse, insbesondere ein Radialgebläse mit halboffenem Laufrad. Der Atemgaspfad führt durch das Gebläse hindurch. Ein Atemgas kann gemäß vorliegender Offenbarung im Bereich des Einlasses aufgenommen und aus dem Auslass ausgestoßen werden.

Im Sinne der vorliegenden Offenbarung soll unter dem ersten Kanal bzw. dem zweiten Kanal prinzipiell ein Durchgang verstanden werden, der eine beliebige Form und/oder Länge aufweisen kann. Dieser Durchgang kann gemäß vorliegender Offenbarung auf beliebige Weise durch Komponenten des Beatmungsgeräts gebildet sein, beispielsweise durch Innenwände, Teile des Gehäuses, Schläuche, etc. Der erste Kanal weist bevorzugt eine Öffnung zur Kammer hin auf, die nachfolgend als eine erste Öffnung bezeichnet werden soll. Der zweite Kanal weist bevorzugt eine Öffnung zur Kammer hin auf, die nachfolgend als eine zweite Öffnung bezeichnet werden soll. Der erste Kanal bzw. der zweite Kanal kann sich gemäß möglichen Ausführungsformen der vorliegenden Offenbarung von unmittelbar angrenzenden Abschnitten des Atemgaspfads, insbesondere der Kammer, unterscheiden, insbesondere hinsichtlich einer Größe seines Querschnitts orthogonal zur Durchflussrichtung. Dabei ist es bevorzugt, wenn sich ein Querschnitt und/oder eine Form des Atemgaspfads an einem Übergang zwischen dem ersten Kanal bzw. dem zweiten Kanal und einem angrenzenden Abschnitt des Atemgaspfads, insbesondere der Kammer, abrupt bzw. sprunghaft verkleinert oder vergrößert.

In einer besonderen Ausführungsform kann der erste bzw. auch der zweite Kanal eine minimale Länge bzw. dreidimensionale Erstreckung ins Volumen bzw. in die Kammer, insbesondere auch nahezu gar keine längliche Erstreckung, aufweisen. Die Kanäle können dazu insbesondere in Schlitz-Form ausgebildet sein. Insbesondere können die Kanäle derart in Schlitz-Form ausgebildet sein, dass der Eintritt in sowie Austritt aus jenen Kanälen wie eine Art Schlitz zwischen vor bzw. hinter dem liegenden Raum und der Kammer ausgebildet sind. Insbesondere kann die jeweilige Länge des jeweiligen Kanals der Dicke der den jeweiligen Kanal umgebenden Wandung entsprechen.

Erfindungsgemäß ist das Gebläse in der Kammer angeordnet. Somit lässt sich eine kompakte Bauform erzielen, bei der das Volumen der Kammer zur Unterbringung des Gebläses genutzt wird. Bei dieser Ausführungsform wurde eine verbesserte Schalldämmung beobachtet. Hierfür gibt es unterschiedliche Erklärungsansätze: Zunächst ist das Gebläse aufgrund der Tatsache, dass es innerhalb der Kammer angeordnet ist, von der Umgebung des Beatmungsgeräts zusätzlich abgeschirmt. Ferner werden von dem Gebläse ausgehende Schallwellen durch die Struktur aus Kammer, erstem Kanal und zweitem Kanal gemäß dem vorangehend beschriebenen Wirkprinzip gedämpft.

Ein weiterer Vorteil dieser Ausführungsform besteht darin, dass durch Konvektion Wärme von dem Gebläse auch äußerlich abgeführt werden kann, anstatt nur innerlich. Das Gebläse ist bevorzugt vollständig in der Kammer angeordnet, wobei Zuleitungen des Gebläses, wie Schläuche und dergleichen, nicht als Teil des Gebläses aufgefasst werden sollen. Diese können sich gemäß möglichen Ausführungsformen der vorliegenden Offenbarung auch außerhalb der Kammer befinden oder beispielsweise abschnittsweise in einer Wandung der Kammer angeordnet sein. Das Gebläse ist vorzugsweise so in der Kammer angeordnet, dass es mehrseitig von einem Atemgas umströmt werden kann, was zur besseren Kühlung dienen kann. Gemäß einer vorteilhaften Variante der vorliegenden Offenbarung ist das Gebläse nicht an einer Wandung der Kammer flächig befestigt und/oder das Gebläse oder ein Abschnitt des Gebläses ist nicht Teil einer Wandung oder mehrerer Wandungen der Kammer. Gemäß vorliegender Offenbarung kann das Gebläse von einer oder mehreren Wandungen der Kammer beabstandet angeordnet sein. Das Gebläse kann beispielsweise durch ein oder mehrere Halteelemente von der Wandung beabstandet gehalten oder gelagert sein. Bevorzugt weist das Gebläse innerhalb der Kammer keine Öffnung zur Aufnahme oder zum Ausstoß des Atemgases auf.

Der Gebläsekörper des Gebläses ist bevorzugt in der Kammer befestigt oder aufgehängt. Es ist besonders bevorzugt, wenn der Gebläsekörper in der Kammer vibrationsdämpfend befestigt ist. Vorzugsweise ist der Gebläsekörper über ein vibrationsdämpfendes Material in der Kammer befestigt. Das vibrationsdämpfende Material kann gemäß vorliegender Offenbarung ein Elastomer sein. Bei dem vibrationsdämpfenden Material handelt es sich bevorzugt um Silikon. Bevorzugt ist der Gebläsekörper über drei Aufhängungspunkte in der Kammer befestigt. Dabei können nach einer Ausführungsform der vorliegenden Offenbarung zwei der Aufhängungspunkte des Gebläsekörpers an Haltern befestigt sein, und ferner kann der dritte Aufhängungspunkt des Gebläsekörpers an einem Druckstutzen befestigt sein. Zwischen den Haltern und dem Gebläsekörper bzw. zwischen dem Druckstutzen und dem Gebläsekörper ist bevorzugt das vibrationsdämpfende Material angeordnet. Die vibrationsdämpfende Aufhängung bzw. Befestigung des Gebläses bewirkt, dass der Gebläsekörper weniger stark vibriert. Ferner werden Vibrationen des Gebläsekörpers nur gedämpft auf das Gehäuse des Beatmungsgeräts übertragen. Dies reduziert Schallemissionen des Beatmungsgeräts zusätzlich.

Die Kammer kann eine beliebige Querschnittsform aufweisen. Sie kann gemäß vorliegender Offenbarung rechteckig sein. Sie muss jedoch nicht eckig sein und kann gemäß einer Ausführungsform der vorliegenden Offenbarung innen rund sein. Sowohl längliche als auch kurze Ausgestaltungsformen der Kammer sind gemäß vorliegender Offenbarung denkbar. Es ist allerdings vorteilhaft, wenn die Kammer ein Volumen von 100 $cm^3$ - 5000 $cm^3$, bevorzugt 500 $cm^3$ - 3000 $cm^3$, besonders bevorzugt 600 - 2000 $cm^3$ oder 100-2000 $cm^3$ aufweist. Es hat sich als besonders vorteilhaft herausgestellt, dass die Kammer ein Volumen von 500-1200 $cm^3$ aufweist.

Bevorzugt wird der Atemgaspfad zwischen dem Einlass und der Kammer zumindest abschnittsweise durch den ersten Kanal gebildet. Der erste Kanal ist folglich zwischen dem Einlass und der Kammer angeordnet. Somit wird ein Atemgas ausgehend von dem Einlass durch den ersten Kanal in die Kammer geleitet. Ferner kann der Atemgaspfad zwischen dem Auslass und der Kammer zumindest abschnittsweise durch den zweiten Kanal gebildet werden. Der zweite Kanal ist somit zwischen dem Auslass und der Kammer angeordnet. Somit kann das Atemgas aus der Kammer durch den zweiten Kanal in Richtung Auslass strömen. Erfindungsgemäß ist vorgesehen, dass der Atemgaspfad zwischen dem zweiten Kanal und dem Auslass durch das Gebläse geführt ist. Ein Atemgas, das aus der Kammer austritt, passiert somit zunächst das Gebläse, bevor es zu dem Auslass gelangt. Das Gebläse kann somit das Atemgas entlang des Atemgaspfads befördern. Es kann alternativ jedoch auch vorgesehen sein, dass das Gebläse entlang des Atemgaspfads zwischen dem Einlass und dem ersten Kanal angeordnet ist.

Gemäß einer besonderen Ausführungsform der vorliegenden Offenbarung mündet der zweite Kanal in eine Zusatzkammer, wobei eine vierte akustische Impedanz der Zusatzkammer von der zweiten akustischen Impedanz des zweiten Kanals abweicht, sodass der durch das Gebläse erzeugte Schall zusätzlich gedämpft wird. Die Zusatzkammer kann eine beliebige Form aufweisen. Es ist vorteilhaft, wenn die Zusatzkammer ein Volumen von 10-200 $cm^3$ aufweist. Als besonders vorteilhaft hat sich ein Volumen von 30-100 $cm^3$ erwiesen. Eine Öffnung des Gebläses ist vorzugsweise unmittelbar oder über einen dritten Kanal mit der Zusatzkammer verbunden. Bei der Öffnung des Gebläses handelt es sich bevorzugt um einen Einlass des Gebläses. Der dritte Kanal kann beispielsweise ein Schlauch oder ein Druckstutzen sein. Gemäß einer Variante der vorliegenden Offenbarung kann der Schlauch oder der Druckstutzen abschnittsweise durch ein schalldämpfendes Material gebildet sein oder über ein schalldämpfendes Material mit dem Gebläse verbunden sein.

Die Kammer und die Zusatzkammer grenzen vorzugsweise aneinander an. Auf diese Weise lässt sich eine kompakte Bauform erzielen. Gemäß einer Variante der vorliegenden Offenbarung ist auf einer Seite einer Wandung des Beatmungsgeräts die Kammer angeordnet und auf einer anderen Seite dieser Wandung ist die Zusatzkammer angeordnet. Es kann gemäß vorliegender Offenbarung vorgesehen sein, dass sowohl der erste Kanal als auch der zweite Kanal durch ebendiese Wandung hindurchgeführt sind. Außerdem kann der dritte Kanal durch diese Wandung hindurchgeführt sein. Es kann ferner vorgesehen sein, dass der Gebläsekörper des Gebläses an ebendieser Wandung befestigt ist. Bei der Wandung handelt es sich bevorzugt um eine Bodenplatte der Kammer.

Gemäß einer alternativen Ausführungsform der vorliegenden Offenbarung ist der zweite Kanal unmittelbar an die Öffnung des Gebläses angeschlossen. Zwischen einem Ausgang des zweiten Kanals und der Öffnung des Gebläses befindet sich folglich keine Zusatzkammer oder kein sonstiger Hohlraum. Es ist gemäß vorliegender Offenbarung möglich, dass der zweite Kanal aus der Kammer herausgeführt ist und an eine außerhalb der Kammer angeordnete Öffnung des Gebläses angeschlossen ist. Es ist alternativ möglich, dass der zweite Kanal vollständig innerhalb der Kammer angeordnet ist und an eine innerhalb der Kammer angeordnete Öffnung des Gebläses angeschlossen ist. Der zweite Kanal ist demgemäß nicht durch eine Wandung aus der Kammer herausgeführt.

Gemäß einer Ausführungsform können sich der erste Kanal und/oder der zweite Kanal in die Kammer erstrecken. Dies bedeutet, dass der erste Kanal und/oder der zweite Kanal in die Kammer hineinragt. Der erste Kanal und der zweite Kanal können gemäß vorliegender Offenbarung durch eine Wandung, welche die Kammer begrenzt, hindurchgeführt sein. Nach einer alternativen Ausgestaltungsform der vorliegenden Offenbarung kann der erste Kanal und/oder der zweite Kanal bereits in der Wandung enden, sodass er überhaupt nicht in die Kammer hineinragt.

Gemäß einer Ausführungsform kann sich der erste Kanal und der zweite Kanal in die Kammer hineinragend erstrecken, wobei der erste Kanal und der zweite Kanal sich vorzugsweise unterschiedlich weit in die Kammer hineinragend erstrecken, unterschiedlich ausgerichtet sind und/oder sich aus unterschiedlichen Seitenwänden in die Kammer hineinragend erstrecken. Gemäß einer vorteilhaften Ausführungsform der vorliegenden Offenbarung sind der erste Kanal und der zweite Kanal parallel zueinander ausgerichtet. Alternativ können der erste Kanal und der zweite Kanal in einem Winkel von 90° zueinanderstehen. Der erste Kanal und der zweite Kanal können jedoch auch einen beliebigen anderen Winkel einschließen. Somit sind unterschiedliche Anordnungen des ersten Kanals und des zweiten Kanals denkbar. Es ist gemäß vorliegender Offenbarung ferner möglich, dass der erste Kanal und der zweite Kanal unterschiedliche Formen aufweisen.

Sowohl eine Position, eine Form als auch eine Dimensionierung des ersten Kanals und des zweiten Kanals können in Abhängigkeit von einer Größe der Kammer, einer Form der Kammer und/oder einer Position des Gebläses gewählt werden. Dies ist vor allem bei Ausführungsformen von Vorteil, bei denen das Gebläse innerhalb der Kammer angeordnet ist. Mit Hilfe einer entsprechenden Anordnung kann außerdem eine verbesserte Strömungsführung innerhalb der Kammer erreicht werden, sodass Strömungsgeräusche verringert werden. Insbesondere sollte angestrebt werden, dass eine Strömungsgeschwindigkeit des Atemgases zwischen dem ersten Kanal und dem zweiten Kanal nicht zu hoch wird. Um dies zu verhindern, sollten die Öffnungen des ersten Kanals und des zweiten Kanals vorzugsweise nicht direkt aufeinander gerichtet werden und/oder die Kanäle sollten sich unterschiedlich weit in die Kammer erstrecken. Dies gilt insbesondere dann, falls der erste Kanal und der zweite Kanal relativ nah beieinander angeordnet sind.

Erfindungsgemäß erstrecken sich der erste Kanal und der zweite Kanal in die Kammer hineinragend, wobei die erste Öffnung des ersten Kanals und die zweite Öffnung des zweiten Kanals so angeordnet sind, dass sich beim Betrieb des Beatmungsgeräts eine Atemgasströmung einstellt, die das innerhalb der Kammer angeordnete Gebläse zumindest abschnittsweise umströmt. Somit wird das Gebläse durch die umströmende Atemgasströmung gekühlt. Um dies zu erreichen, können der erste und der zweite Kanal entsprechend dimensioniert bzw. ausgerichtet sein. Der Gebläsekörper befindet sich gemäß dieser Variante der vorliegenden Offenbarung vorzugsweise zwischen der ersten Öffnung des ersten Kanals und der zweiten Öffnung des zweiten Kanals.

Der erste Kanal und/oder der zweite Kanal erstreckt sich vorzugsweise außerhalb der Kammer aus der Wandung der Kammer heraus. Der erste Kanal bzw. der zweite Kanal ist somit außenseitig aus der Kammer herausgeführt. Der erste Kanal und/oder der zweite Kanal können sich bei dieser Ausführungsform gleichwohl auch in die Kammer hinein erstrecken. Somit sind kompakte Bauformen möglich, zum Beispiel in dem Fall, dass die Kammer vergleichsweise flach ausgeführt sein soll. Dann kann ein Abschnitt des ersten Kanals und/oder des zweiten Kanals außerhalb der Kammer angeordnet sein. Gemäß vorliegender Offenbarung können der erste Kanal und der zweite Kanal außerhalb der Kammer unterschiedliche Längen aufweisen, unterschiedlich dimensioniert sein und/oder unterschiedlich ausgerichtet sein. Nach vorteilhaften Ausgestaltungsformen der vorliegenden Offenbarung bildet der erste Kanal außerhalb der Kammer den Einlass des Beatmungsgeräts. Gemäß vorliegender Offenbarung kann außerdem vorgesehen sein, dass der zweite Kanal außerhalb der Kammer den Auslass des Beatmungsgeräts bildet.

Gemäß einer Ausführungsform können der erste Kanal und/oder der zweite Kanal einen gekrümmten oder einen gewellten Verlauf aufweisen. Der erste Kanal bzw. der zweite Kanal mit gekrümmtem Verlauf ist vorzugsweise zur Seite gekrümmt, beispielsweise bananenförmig bzw. bogenförmig. Dadurch ist die Öffnung des ersten Kanals bzw. des zweiten Kanals in die Kammer bevorzugt seitlich ausgerichtet. Somit kann vermieden werden, dass eine aus dem Kanal austretende Atemgasströmung senkrecht auf eine Wandung der Kammer auftrifft. Es ist strömungstechnisch vorteilhaft, dass dies vermieden wird, um ein Strömungsrauschen zu reduzieren. Ein gewellter Verlauf birgt bei einer entsprechenden Ausrichtung der Öffnung des ersten Kanals bzw. des zweiten Kanals einen entsprechenden Vorteil, ferner kann der erste Kanal bzw. der zweite Kanal um Hindernisse innerhalb der Kammer herum geführt sein.

Gemäß einer Ausführungsform können der erste Kanal und/oder der zweite Kanal eine Querschnittsfläche orthogonal zu einer Längsrichtung des ersten Kanals bzw. des zweiten Kanals aufweisen, die gekrümmt ausgebildet ist. Die Querschnittsfläche kann gemäß vorliegender Offenbarung halbmondförmig sein oder ein in sonstiger Weise gekrümmtes Profil aufweisen. Auf diese Weise lassen sich strömungstechnische Vorteile erreichen. Beispielsweise kann die Atemgasströmung um ggf. in der Kammer angeordnete Bauteile herumgeführt werden, um ein Strömungsrauschen zu vermindern.

Der erste Kanal und/oder der zweite Kanal können gemäß einer Ausführungsform ein Volumen mit rechteckigem Querschnitt, insbesondere ein im Wesentlichen quaderförmiges Volumen bilden. Gleichwohl sind aber auch abweichende Formen möglich. Das Volumen weist bevorzugt eine Tiefe von 1-50 mm, bevorzugt 1-20 mm, besonders bevorzugt 3-7 mm, eine Breite von 5-100 mm, bevorzugt 20-60 mm und eine Länge von 1-100 mm, bevorzugt 20-60 mm, auf. Dadurch ergibt sich eine deutlich verminderte Schallabstrahlung des Beatmungsgeräts. Dies gilt insbesondere dann, wenn die Kammer bzw. die Zusatzkammer wie vorangehend definiert dimensioniert ist. Die Länge des Volumens ist als eine Ausdehnung des Volumens in Strömungsrichtung zu verstehen. Die Breite und die Tiefe des Volumens spannen eine Querschnittsfläche des Volumens auf, die senkrecht zu der Strömungsrichtung ausgerichtet ist.

Gemäß einer weiteren Ausführungsform der vorliegenden Offenbarung ist/sind der erste Kanal und/oder der zweite Kanal so gestaltet, dass er sich in Richtung einer Öffnung des Kanals in die Kammer verengt oder erweitert. Vorzugsweise ist der Öffnungswinkel nicht größer als 20°. So kann ein Verlauf der Atemgasströmung angepasst werden, um eine Verminderung des Strömungsrauschens zu erzielen. Ferner hat diese Ausgestaltungsform der vorliegenden Offenbarung fertigungstechnische Vorteile.

Es ist vorteilhaft, wenn der erste Kanal und/oder der zweite Kanal ausgerundete Innenkanten aufweist/aufweisen. Dies hat fertigungstechnische Vorteile. Es ist aber alternativ auch möglich, dass der erste Kanal und/oder der zweite Kanal eckige Innenkanten aufweist/aufweisen, insbesondere Innenkanten, bei denen Wandstücke in einem Winkel von 90° zueinander ausgerichtet sind. Gemäß vorliegender Offenbarung kann ferner vorgesehen sein, dass in dem ersten Kanal und / oder in dem zweiten Kanal Rippen, insbesondere Längsrippen, angeordnet sind, wodurch sich strömungs- und fertigungstechnische Vorteile ergeben können.

In einer vereinfachten Darstellung der tatsächlichen Geometrien weisen die Kanäle oder Schlitze bedingt durch ihre Tiefe und ihre Breite bestimmbare Querschnitte und Umfänge auf. Beispielsweise kann der Querschnitt der Kanäle rechteckig oder oval mit einem hohen Seitenverhältnis sein. Andere Formen sind ebenfalls vorstellbar und die Querschnitte können eine insgesamt gekrümmte oder eckige Form oder eine andere unregelmäßige Form aufweisen. Der erste Kanal und der zweite Kanal können identische oder voneinander abweichende Querschnittsformen aufweisen. Die Querschnittsform ist senkrecht zu der zu der Strömungsrichtung ausgerichtet. Die Querschnittsform der Kanäle kann zumindest abschnittsweise ausgewählt sein aus den Formen: rechteckig, schlitzförmig, sichelförmig, oval, Kreuz-förmig, X-förmig, Stern-förmig, runder oder eckiger Ringspalt, L-förmig, Hantel-förmig, U-förmig, V-förmig, T-förmig, Ellipsen-förmig, Trapez-förmig, rund. Die Querschnittsformen des ersten Kanals und des zweiten Kanals können identisch oder unterschiedlich ausgebildet sein. In bevorzugten Ausführungsformen können die Kanäle eine im Wesentlichen glatte Innenfläche aufweisen.

Die Querschnittsformen der Kanäle sollten derart gewählt sein, dass sie jeweils ihre akustischen Anforderungen erfüllen. Dafür ist es vorteilhaft, wenn die Kanäle eine möglichst geringe Tiefe aufweisen und dennoch ein bestimmtes Volumen fassen können. Bezüglich des zweiten Kanals ist zu bedenken, dass er benachbart zum Saugstutzen des Gebläses angeordnet ist bzw. in die Ansaugkammer des Gebläses - die Zusatzkammer - mündet. Am Gebläse sollte vorteilhaft eine möglichst symmetrische Strömung ankommen. Aus diesem Grund ist es vorteilhaft, wenn die Geometrie des zweiten Kanals möglichst symmetrisch in die Zusatzkammer übergeht. Die Querschnittsform des zweiten Kanals ist somit bevorzugt möglichst rechteckig bzw. schlitzförmig mit einer geringen Tiefe.

In Bezug auf den ersten Kanal ist es wichtiger, dass in Strömungsrichtung hinter dem ersten Kanal eine freie Länge ist, so dass die Strömung aus dem ersten Kanal frei in die Kammer strömt und nicht direkt auf eine Wand trifft. Die Geometrie des ersten Kanals ist bezogen auf die Strömung weniger kritisch und kann variabler gewählt sein. Die Querschnittsform des ersten Kanals kann ebenso wie der zweite Kanal schlitzförmig sein. In bevorzugten Ausführungsformen kann die Querschnittsform des ersten Kanals aber auch derart gewählt werden, dass eine kompaktere Bauweise realisierbar ist. Dies kann durch Geometrien mit einer geringeren Breite erreicht werden. Es ist hervorzuheben, dass der erste Kanal und der zweite Kanal nicht Teil eines Druckdifferenzsensors sind.

Beispielsweise sind mindestens zwei solche Kanäle in dem Atemgaspfad angeordnet, wobei diese Kanäle in Reihe und durch eine Zwischenkammer oder Zusatzkammer verbunden sein können. Die beiden Kanäle können im Wesentlichen den gleichen Querschnitt haben, oder die Querschnitte können unterschiedlich sein.

Die relative Größe der Querschnittsflächen und der entsprechenden Umfänge kann durch das

Verhältnis **R** des
Umfangs **P** im Quadrat zur
Fläche **A** charakterisiert werden:

$$R = P^2/A$$

Dieses Verhältnis **R** hat für zumindest einen Kanal oder Schlitz vorzugsweise einen Wert von über 14, bevorzugt 16 oder mehr, besonders bevorzugt 20 oder mehr, besonders bevorzugt 25 oder mehr, stärker bevorzugt 30 oder mehr, beispielsweise auch 30, 35, 40, 45 oder 50. Laborversuche für verschiedene R-Werte sind in der folgenden Tabelle angegeben:

| Kanaltyp | Schalldämpfung | $R = P^2/A$ |
|----------|----------------|-------------|
| K1 | -- | 12 - 14 |
| K2 | + | 16 - 20 |
| K3 | + | 21 - 25 |
| K4 | + | >25 |

(fortgesetzt)

| Kanaltyp | Schalldämpfung | $R = P^2/A$ |
|----------|----------------|-------------|
| K5 | - | >40 |

In einer bevorzugten Ausführungsform ist der Übergang zwischen einem oder beiden Kanälen und der Kammer abrupt, was bedeutet, dass es eine deutliche geometrische Diskontinuität gibt.

In einigen Ausführungsformen haben die Kanäle beispielsweise breitere Endabschnitte. Dies bietet den Vorteil einer Absenkung des Strömungsverlustes.

Eine Filterkammer zum Unterbringen einer entfernbaren Filterkomponente kann im Atemgaspfad unmittelbar stromaufwärts eines Kanals positioniert sein.

Einer oder mehrere Kanäle können in Strömungsrichtung im Wesentlichen gerade oder gekrümmt verlaufen.

Einer oder beide Kanäle können in mehrere parallel laufende Teilkanäle aufgeteilt werden. Der Querschnittsumfang jedes Kanals wird dann als Summe der Querschnittsumfang seiner Unterkanäle bewertet. In ähnlicher Weise wird die Querschnittsfläche als Summe der Querschnittsflächen seiner Teilkanäle bewertet.

In einer Ausführungsform weist zumindest ein Kanal eine Querschnittsfläche A und einen Umfang P auf, die durch das Verhältnis **R,** des Umfangs **P** im Quadrat, zur Fläche **A** charakterisiert werden entsprechend der Formel $R = P^2/A$

wobei dieses Verhältnis **R** einen Wert von über 14, bevorzugt 16 oder über 16, besonders bevorzugt 20 oder über 20, stärker bevorzugt 30 oder über 30 einnimmt.

In den Zeichnungen sind vorteilhafte Ausführungsformen der vorliegenden Offenbarung dargestellt. Dabei zeigt:

Fig. 1      eine Außenansicht einer ersten Ausführungsform des Beatmungsgeräts gemäß vorliegender Offenbarung,

Fig. 2      eine Schnittansicht der ersten Ausführungsform des Beatmungsgeräts gemäß vorliegender Offenbarung,

Fig. 3      eine Teilansicht der ersten Ausführungsform des Beatmungsgeräts gemäß vorliegender Offenbarung,

Fig. 4      eine Teilansicht einer zweiten Ausführungsform des Beatmungsgeräts gemäß vorliegender Offenbarung, wobei ein erster Kanal des Beatmungsgeräts einen gekrümmten Verlauf aufweist,

Fig. 5      eine Teilansicht einer dritten Ausführungsform des Beatmungsgeräts gemäß vorliegender Offenbarung, wobei ein erster Kanal und ein zweiter Kanal des Beatmungsgeräts Querschnittsflächen aufweisen, die gekrümmt ausgebildet sind,

Fig. 6      eine Teilansicht einer vierten Ausführungsform des Beatmungsgeräts gemäß vorliegender Offenbarung, wobei ein erster Kanal und ein zweiter Kanal des Beatmungsgeräts so ausgestaltet sind, dass sie sich in Richtung ihrer Öffnungen in eine Kammer des Beatmungsgeräts erweitern,

Fig. 7      eine Teilansicht einer fünften Ausführungsform des Beatmungsgeräts gemäß vorliegender Offenbarung, wobei ein erster Kanal des Beatmungsgeräts so ausgestaltet ist, dass er sich in Richtung seiner Öffnung in eine Kammer des Beatmungsgeräts erweitert, wobei der erste Kanal zusätzlich gekrümmt ausgebildet ist,

Fig. 8      eine Teilansicht einer sechsten Ausführungsform des Beatmungsgeräts gemäß vorliegender Offenbarung, wobei ein erster Kanal und ein zweiter Kanal des Beatmungsgeräts Längsrippen aufweisen, und

Fig. 9      eine Teilansicht einer siebten Ausführungsform des Beatmungsgeräts gemäß vorliegender Offenbarung, wobei ein erster Kanal und ein zweiter Kanal des Beatmungsgeräts sich außerhalb einer Kammer des Beatmungsgeräts aus einer Wandung der Kammer erstrecken.

Fig. 10/11      schematische Ansichten verschiedener Ausführungsformen der Querschnittsformen des ersten und des zweiten Kanals.

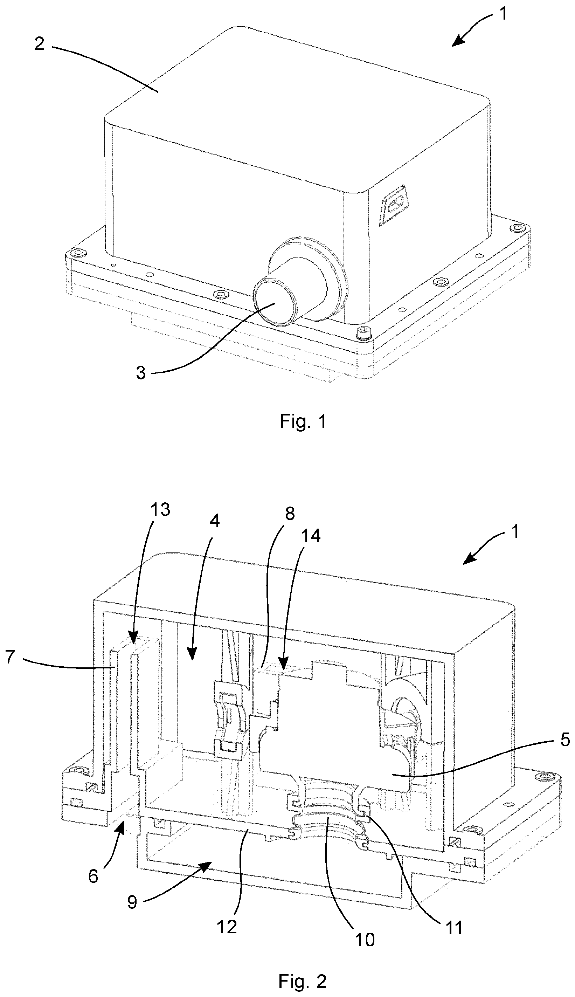

Fig. 1 zeigt eine Außenansicht einer ersten Ausführungsform des Beatmungsgeräts 1 gemäß vorliegender Offenbarung. Das Beatmungsgerät 1 weist ein Gehäuse 2 auf. Aus einem Auslass 3 stößt das Beatmungsgerät 1 ein Atemgas aus. Das Beatmungsgerät 1 weist ferner einen Einlass auf, der auf einer Unterseite des Beatmungsgeräts 1 angeordnet und in Fig. 1 nicht sichtbar ist.

Fig. 2 zeigt eine Schnittansicht der ersten Ausführungsform des Beatmungsgeräts 1 gemäß vorliegender Offenbarung. Das Beatmungsgerät 1 bildet eine Kammer 4. In der Kammer 4 ist ein Gebläse 5 des Beatmungsgeräts 1 angeordnet. Bei dem Gebläse 5 handelt es sich um ein Radialgebläse.

Das Beatmungsgerät 1 weist einen Einlass 6 auf, durch den das Atemgas in das Beatmungsgerät 1 eintreten kann. Das durch den Einlass 6 in das Beatmungsgerät 1 eintretende Atemgas folgt innerhalb des Beatmungsgeräts 1 folgendem Atemgaspfad: Zunächst durchfließt das Atemgas einen ersten Kanal 7 des Beatmungsgeräts 1. Anschließend tritt das Atemgas in die Kammer 4 ein. Das Atemgas durchströmt die Kammer 4 und tritt nachfolgend in einen zweiten Kanal 8 ein. Durch den zweiten Kanal 8 gelangt das Atemgas in eine Zusatzkammer 9. Aus der Zusatzkammer 9 wird das Atemgas durch einen Stutzen 10 des Beatmungsgeräts 1 von dem Gebläse 5 angesaugt. Das Gebläse 5 befördert das Atemgas weiter, sodass es schließlich aus dem in Fig. 2 nicht dargestellten Auslass des Beatmungsgeräts 1 austritt.

Ein Endabschnitt 11 des Stutzens 10 besteht aus Silikon. Somit ist das Gebläse 5 vibrationsdämpfend mit einer Bodenplatte 12 der Kammer 4 verbunden.

Eine erste akustische Impedanz des ersten Kanals 7 und eine zweite akustische Impedanz des zweiten Kanals 8 weichen von einer dritten akustischen Impedanz der Kammer 4 ab. An einem Übergang von einer ersten Öffnung 13 des ersten Kanals 7 in die Kammer 4 und einem Übergang von einer zweiten Öffnung 14 des zweiten Kanals 8 in die Kammer 4 treten deshalb Schallreflektionen auf. Somit wird ein durch das Gebläse 5 erzeugter Schall gedämpft. Betriebsgeräusche des Beatmungsgeräts 1 werden dadurch vermindert.

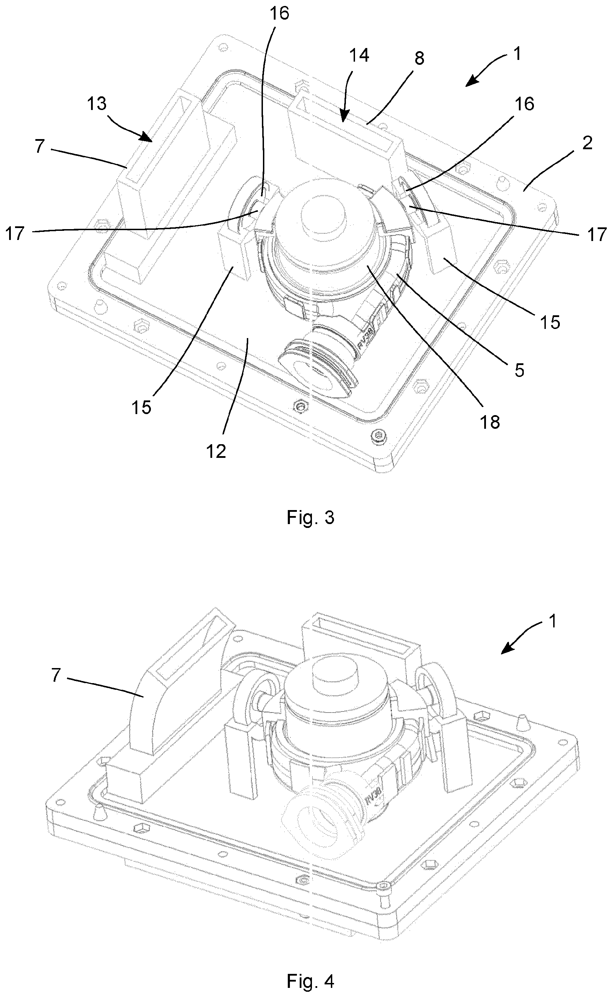

Fig. 3 zeigt eine Teilansicht der ersten Ausführungsform des Beatmungsgeräts 1 gemäß vorliegender Offenbarung. Bei dieser Teilansicht sowie allen folgenden Teilansichten des Beatmungsgeräts 1 ist ein oberer Gehäuseabschnitt nicht dargestellt, sodass ein Inneres der Kammer besser sichtbar ist.

Der erste Kanal 7 und der zweite Kanal 8 sind zueinander rechtwinklig ausgerichtet. Vorliegend sind der erste Kanal 7 und der zweite Kanal 8 so angeordnet, dass sich zwischen ihren Öffnungen 13 und 14 keine Hindernisse befinden. Dies dient der Vermeidung eines Strömungsrauschens.

Bei der vorliegenden Ausführungsform sind an der Bodenplatte 12 zwei Halter 15 angebracht, die der zusätzlichen Befestigung des Gebläses 5 in dem Beatmungsgerät 1 dienen. In Endbereichen weisen die Halter 15 Silikonlager 16 auf, in denen Haltestifte 17 des Gebläses 5 sitzen. Die Haltestifte 17 sind mit einem Gebläsekörper 18 des Gebläses 5 verbunden. Das Gebläse 5 ist somit sowohl an den Haltern 15 als auch an dem Stutzen 10 vibrationsdämpfend befestigt. Dies hat eine zusätzliche schalldämpfende Wirkung und vermindert insbesondere eine Schallübertragung durch Vibrationen von dem Gebläsekörper 5 auf das Gehäuse 2 des Beatmungsgeräts 1.

Fig. 4 zeigt eine Teilansicht einer zweiten Ausführungsform des Beatmungsgeräts 1 gemäß vorliegender Offenbarung, wobei ein erster Kanal 7 des Beatmungsgeräts 1 einen gekrümmten Verlauf aufweist. Aufgrund des gekrümmten Verlaufs trifft eine aus dem ersten Kanal 7 austretende Atemgasströmung nicht senkrecht auf eine (hier in Fig. 4 nicht dargestellte) obere Wandung des Beatmungsgeräts 1 auf. Dies ist strömungstechnisch vorteilhaft, da somit ein Strömungsrauschen vermindert wird.

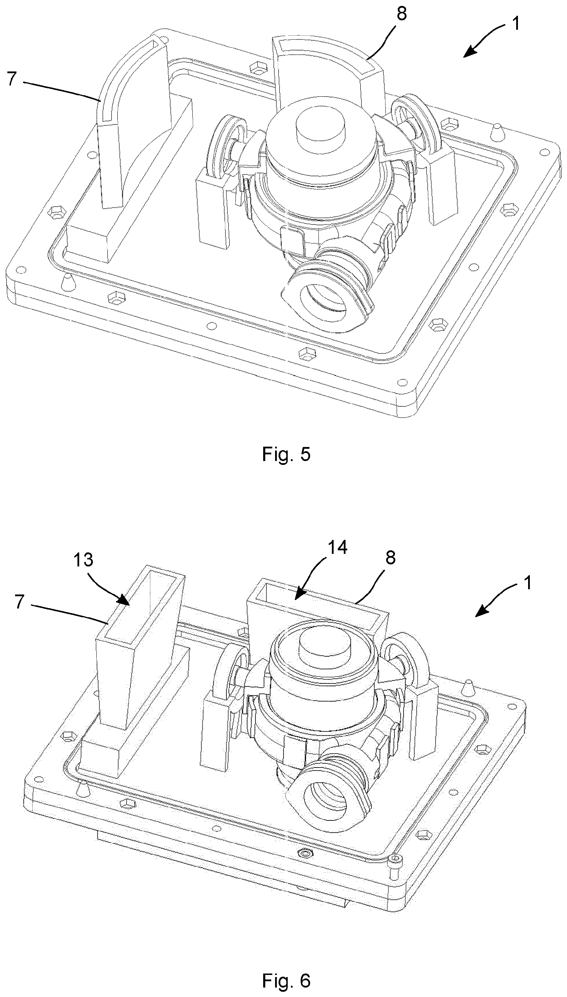

Fig. 5 zeigt eine Teilansicht einer dritten Ausführungsform des Beatmungsgeräts 1 gemäß vorliegender Offenbarung, wobei ein erster Kanal 7 und ein zweiter Kanal 8 des Beatmungsgeräts 1 Querschnittsflächen aufweisen, die gekrümmt ausgebildet sind. Der erste Kanal 7 und der zweite Kanal 8 ragen unterschiedlich weit in eine Kammer des Beatmungsgeräts 1 hinein. Auf diese Weise kann ein vorteilhafter Verlauf der Atemgasströmung in der Kammer erzielt werden, um ein Strömungsrauschen zu vermindern.

In Anlehnung an das in Fig. 5 dargestellte Ausführungsbeispiel können auch weitere Ausführungsbeispiele vorgesehen sein, die eine abweichende Form einer Krümmung der gekrümmt ausgebildeten Kanäle umfassen. So ist, nur um ein Beispiel zu nennen, auch ein S-förmiger Querschnitt, im Sinne eines "S-Schlags", als Krümmung der Kanäle denkbar.

Es ist ebenso möglich, dass einer der beiden Kanäle eine Form einer Krümmung aufweist, während der andere der beiden Kanäle eine abweichende Form einer Krümmung aufweist.

Fig. 6 zeigt eine Teilansicht einer vierten Ausführungsform des Beatmungsgeräts 1 gemäß vorliegender Offenbarung, wobei ein erster Kanal 7 und ein zweiter Kanal 8 des Beatmungsgeräts 1 so ausgestaltet sind, dass sie sich in Richtung ihrer Öffnungen 13 und 14 in eine Kammer des Beatmungsgeräts 1 erweitern. Dies dient der vorteilhaften Anpassung der Atemgasströmung, um eine Verminderung des Strömungsrauschens zu erzielen.

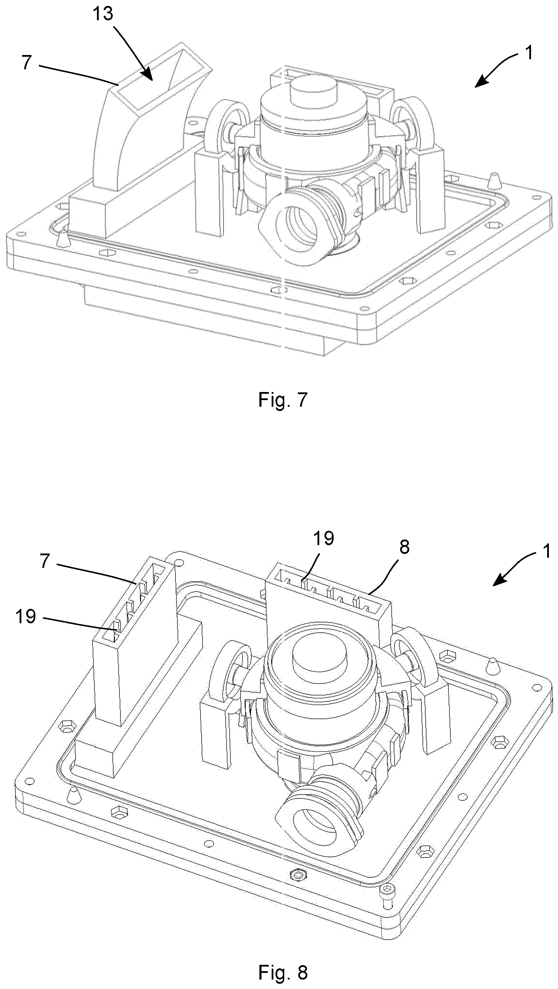

Fig. 7 zeigt eine Teilansicht einer fünften Ausführungsform des Beatmungsgeräts 1 gemäß vorliegender Offenbarung, wobei ein erster Kanal 7 des Beatmungsgeräts 1 so ausgestaltet ist, dass er sich in Richtung einer ersten Öffnung 13 des ersten Kanals 7 in die Kammer erweitert, und wobei der erste Kanal 7 zusätzlich gekrümmt ausgebildet ist. Somit werden Vorteile der dritten Ausführungsform und der vierten Ausführungsform kombiniert, um eine Verminderung des Strömungsrauschens zu erzielen.

Fig. 8 zeigt eine Teilansicht einer sechsten Ausführungsform des Beatmungsgeräts 1 gemäß vorliegender Offenbarung, wobei ein erster Kanal 7 und ein zweiter Kanal 8 des Beatmungsgeräts 1 Längsrippen 19 aufweisen. Dies hat strömungs- und fertigungstechnische Vorteile.

In Anlehnung an das in Fig. 8 dargestellte Ausführungsbeispiel können auch weitere Ausführungsbeispiele vorgesehen sein, die eine abweichende Form der in Fig. 8 noch als Längsrippen 19 ausgebildete Rippen umfassen. So sind, nur um ein Beispiel zu nennen, auch Querrippen denkbar. Ebenfalls ist es möglich, abweichende bzw. kombinierte Formen an Rippen, seien es Querrippen oder Längsrippen oder auch die Kombination aus Querrippen und Längsrippen, vorzusehen. Um ein Beispiel zu nennen, so können die Rippen auch in Tragflügel-Form ausgebildet sein. Weiterhin ist es möglich, die Rippen lediglich in einem Teil der länglichen Erstreckung des entsprechenden Kanals vorzusehen und die Rippen demnach nicht auf voller Länge des Kanals auszubilden.

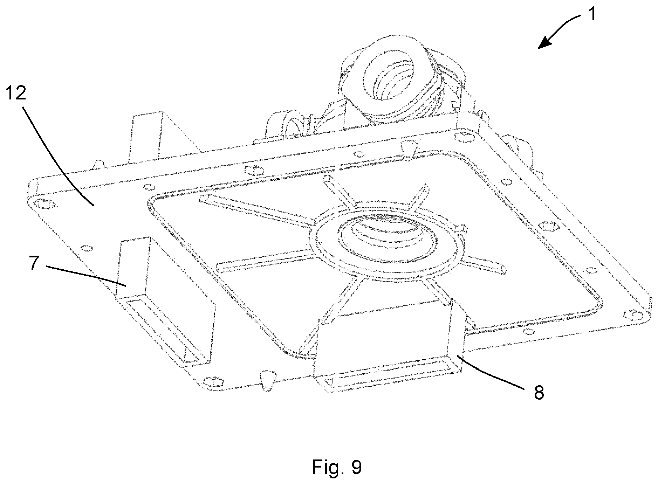

Fig. 9 zeigt eine Teilansicht einer siebten Ausführungsform des Beatmungsgeräts 1 gemäß vorliegender Offenbarung, wobei ein erster Kanal 7 und ein zweiter Kanal 8 des Beatmungsgeräts 1 sich außerhalb einer Kammer des Beatmungsgeräts 1 aus einer Wandung der Kammer erstrecken, wobei es sich bei der Wandung um eine Bodenplatte 12 der Kammer handelt. Der erste Kanal 7 und der zweite Kanal 8 ragen somit beidseitig aus der Bodenplatte 12 hervor. Dies erlaubt eine flachere Bauform der Kammer des Beatmungsgeräts 1.

In Anlehnung an das in Fig. 2 dargestellte Ausführungsbeispiel mit der Zusatzkammer 9 sowie an das in Fig. 9 dargestellte Ausführungsbeispiel mit sich außerhalb einer Kammer (etwa der Kammer 4 gemäß Fig. 2) erstreckenden Kanälen können auch weitere Ausführungsbeispiele vorgesehen sein, die zwar einen ersten Kanal, der in die das Gebläse aufweisende Kammer mündet (etwa Kammer 4 mit Gebläse 5 wie in Fig. 2), aufweist, jedoch sodann der zweite Kanal lediglich in der Zusatzkammer (etwa in der unterhalb des Gebläses angeordneten Zusatzkammer) angeordnet ist. Auch weitere Kanäle können bei einer solchen Ausführungsform in der Zusatzkammer angeordnet sein. Ferner ist es denkbar, dass beispielsweise der erste Kanal nur in Schlitz-Form ausgebildet ist und lediglich eine Art Schlitz bzw. Durchbruch in die das Gebläse aufweisende Kammer darstellt.

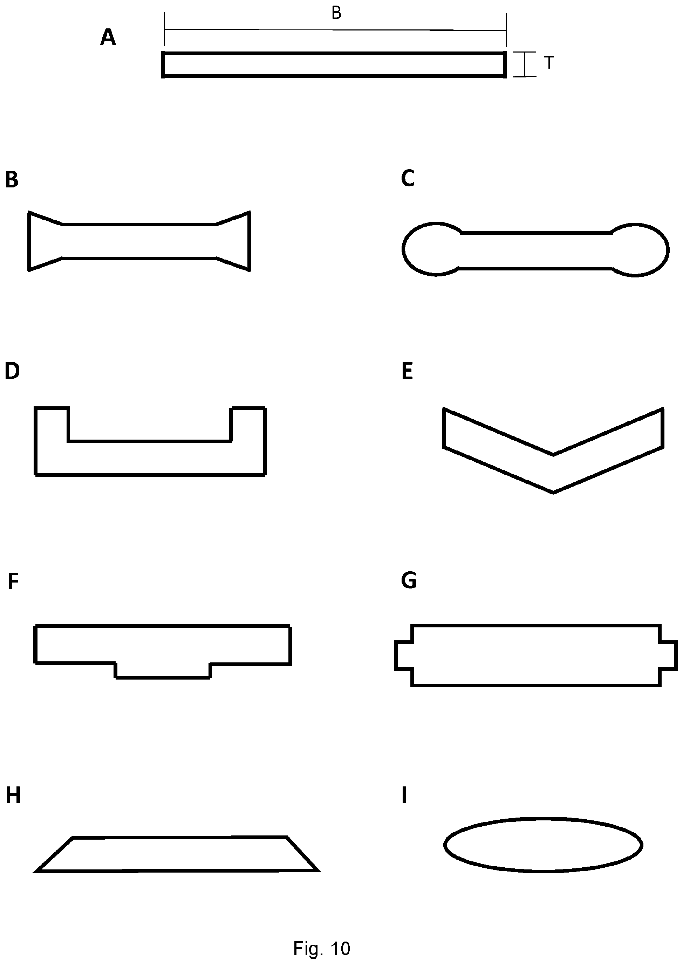

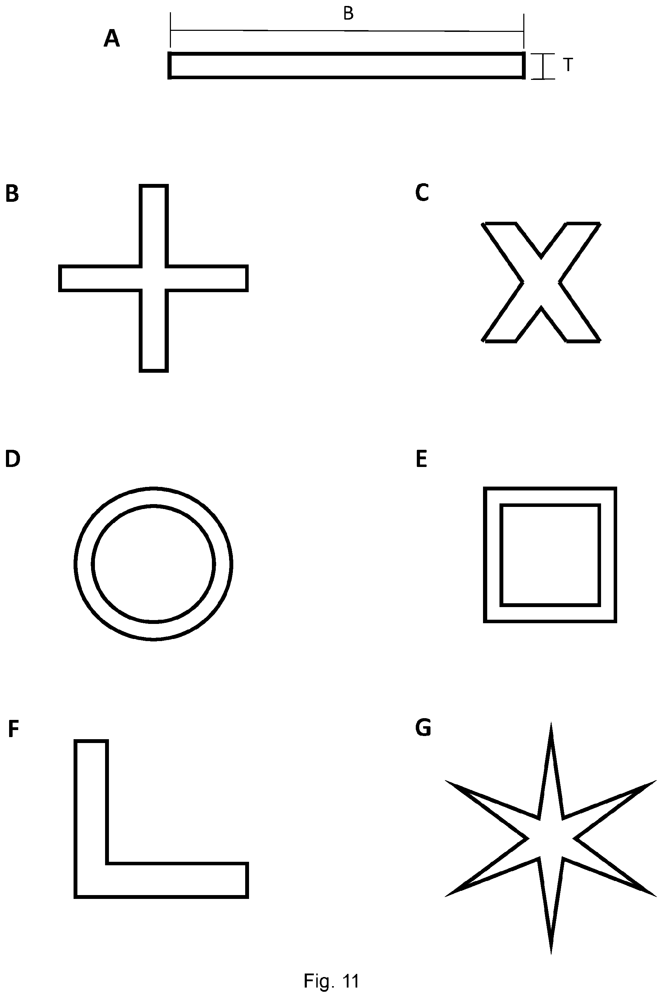

Fig. 10 und Figur 11 zeigen schematische Ansichten verschiedener Ausführungsformen der Querschnittsformen des ersten und des zweiten Kanals. Gezeigt ist die Querschnittsfläche, die orthogonal zur Längsrichtung des Kanals ist. Erster Kanal und zweiter Kanal können identische Querschnittsformen aufweisen oder voneinander abweichende Querschnittsformen.

In Figur 10 sind beispielhafte Ausführungsformen des Querschnittes gezeigt, die für den ersten und den zweiten Kanal geeignet sind. Fig. 10A zeigt eine rechteckige Querschnittsform mit einer Tiefe T und einer Breite B. Die Tiefe T kann um ein Vielfaches geringer sein als die Breite B, so dass der jeweilige Kanal Schlitzförmig ausgebildet ist. Der Kanal kann zusätzlich gekrümmt ausgebildet sein (hier nicht gezeigt, siehe Fig. 5), so dass sich eine sichelförmige Querschnittsform ergibt.

Neben einer rechteckigen Querschnittsform sind auch andere Querschnittsformen denkbar wie in den Figuren 10B-I exemplarisch gezeigt. Fig. 10B und 10C zeigen Hantel-förmige Querschnittsformen. Unter einer hantel-förmigen Querschnittsform kann verstanden werden, dass der Kanal im Querschnitt schlitzförmig ist und Erweiterungen an mindestens einem, bevorzugt an beiden Enden aufweist. Die Erweiterungen können eckig (siehe Fig. 10B) oder rund ausgebildet sein (siehe Fig. 10C). Der Vorteil von Hantel-förmigen Querschnittsformen ist, dass durch die Erweiterungen an den Enden das Strömungsprofil gleichmäßiger ausfällt als bei einem Schlitz ohne Erweiterungen. Dies kann durch den größeren Strömungsquerschnitt an den jeweiligen Enden realisiert sein, der einen geschwindigkeitsmindernden Reibungseffekt an den Enden ausgleichen kann. Fig. 10 D zeigt eine U-förmige Querschnittsform, die durch die Erweiterung an den Enden ähnliche Vorteile wie zuvor genannt aufweisen kann. Weitere mögliche Querschnittsformen können eine V-Form (Fig. 10E), eine T-Form (Fig. 10F), eine Doppel-T-Form (Fig. 10 G), ein Trapez (Fig. 10H) oder eine Ellipse (Fig. 10I) sein.

In Figur 11 sind beispielhafte Ausführungsformen des Querschnittes gezeigt, die in alternativen Ausführungsbeispielen insbesondere für den ersten Kanal geeignet und vorteilhaft sein können. Neben der reinen rechteckigen Schlitzform (Fig. 11A) sind auch andere Querschnittsformen denkbar wie in den Figuren 11B-G exemplarisch gezeigt. Fig. 11B zeigt eine Kreuz-förmige, Fig. 11C eine x-förmige, Fig. 11F eine L-förmige und Fig. 11G eine Stern-förmige Querschnittsform. Bei diesen Querschnittsformen kann der Bauraum durch eine insgesamt geringere Breite B minimiert werden.

In Fig. 11D und 11E ist ein runder bzw. ein eckiger Ringspalt gezeigt. Ein dreieckig oder mehreckig ausgebildeter Ringspalt ist auch denkbar (nicht gezeigt). Diese Querschnittsformen zeichnet aus, dass die Strömung hierbei zwischen dem äußeren Kreis bzw. Viereck und dem inneren Kreis bzw. Viereck verläuft. Aus technischer Sicht entsprechen derartige Formen einem "aufgewickeltem Schlitz", was ebenfalls eine deutliche Einsparung des Bauraumes mit sich bringt.

Sämtliche in Fig. 11B-G dargestellten Geometrien zeichnet jeweils aus, dass der entsprechende Kanal eine kompaktere Bauweise als die Schlitzform gemäß Fig. 11A bei gleichbleibender Querschnittsfläche aufweist. Das Gesamtvolumen, gebildet durch die Tiefe, die Breite und die Länge der Kanäle ist bevorzugt bei allen Querschnittsformen gleich.

Die Länge des Kanals kann unbeeinflusst von den dargestellten möglichen Querschnittsformen gemäß der Figuren 10 und 11 sein. Die Länge des Kanals kann 1-100 mm, bevorzugt 20-60 mm, besonders bevorzugt 40mm betragen. Wichtig bei allen Querschnittsformen ist die Tiefe T. Diese sollte zumindest bei der am Schlitzähnlichsten Teilform gleich sein, nämlich 1-20 mm, bevorzugt 3-7 mm, besonders bevorzugt 5 mm. Die Querschnittsfläche, gebildet durch die Tiefe und die Breite der Kanäle, ist bevorzugt bei allen Querschnittsformen gleich. Die Querschnittsfläche kann 5-2000 mm$^2$, bevorzugt 60-420 mm$^2$, besonders bevorzugt 200 mm$^2$ betragen.

**Bezugszeichenliste**

1    Beatmungsgerät

2 Gehäuse
3 Auslass
4 Kammer
5 Gebläse
6 Einlass
7 Erster Kanal
8 Zweiter Kanal
9 Zusatzkammer
10 Stutzen
11 Endabschnitt
12 Bodenplatte
13 Erste Öffnung
14 Zweite Öffnung
15 Halter
16 Silikonlager
17 Haltestift
18 Gebläsekörper
19 Längsrippe

**Patentansprüche**

1. Beatmungsgerät (1) mit einem Einlass (6), mit einem Auslass (3) und mit einem Gebläse (5), wobei zwischen dem Einlass (6) und dem Auslass (3) ein Atemgaspfad gebildet ist, der durch das Gebläse (5) geführt ist, wobei entlang des Atemgaspfads eine Kammer (4), ein in die Kammer (4) mündender erster Kanal (7) und ein in die Kammer (4) mündender zweiter Kanal (8) angeordnet ist, und wobei eine erste akustische Impedanz des ersten Kanals (7) und eine zweite akustische Impedanz des zweiten Kanals (8) von einer dritten akustischen Impedanz der Kammer (4) abweichen, sodass ein durch das Gebläse (5) erzeugter Schall gedämpft wird, wobei das Gebläse (5) in der Kammer (4) angeordnet ist, wobei sich der erste Kanal (7) und der zweite Kanal (8) in die Kammer (4) hineinragend erstrecken, wobei eine erste Öffnung (13) des ersten Kanals (7) und eine zweite Öffnung (14) des zweiten Kanals (8) so angeordnet sind, dass sich beim Betrieb des Beatmungsgeräts (1) eine Atemgasströmung einstellt, die das Gebläse (5) zumindest abschnittsweise umströmt, wobei der Atemgaspfad zwischen dem zweiten Kanal (8) und dem Auslass (3) durch das Gebläse (5) geführt ist.

2. Beatmungsgerät (1) nach Anspruch 1, wobei ein Gebläsekörper (18) des Gebläses (5) in der Kammer (4) vibrationsdämpfend befestigt ist.

3. Beatmungsgerät (1) nach einem der vorhergehenden Ansprüche, wobei die Kammer (4) ein Volumen von 100 cm$^3$ - 5000 cm$^3$, bevorzugt 500 cm$^3$ - 3000 cm$^3$, besonders bevorzugt 500 - 1200 cm$^3$ aufweist.

4. Beatmungsgerät (1) nach einem der vorhergehenden Ansprüche, wobei der Atemgaspfad zwischen dem Einlass (6) und der Kammer (4) zumindest abschnittsweise durch den ersten Kanal (7) gebildet ist.

5. Beatmungsgerät (1) nach einem der vorhergehenden Ansprüche, wobei der Atemgaspfad zwischen dem Auslass (3) und der Kammer (4) zumindest abschnittsweise durch den zweiten Kanal (8) gebildet ist.

6. Beatmungsgerät (1) nach einem der vorhergehenden Ansprüche, wobei der zweite Kanal (8) in eine Zusatzkammer (9) mündet, wobei eine vierte akustische Impedanz der Zusatzkammer (9) von der zweiten akustischen Impedanz des zweiten Kanals (8) abweicht, sodass der durch das Gebläse (5) erzeugte Schall zusätzlich gedämpft wird, wobei die Zusatzkammer (9) ein Volumen von 10 cm$^3$ - 200 cm$^3$, bevorzugt 30 cm$^3$ - 100 cm$^3$, aufweist.

7. Beatmungsgerät (1) nach einem der vorhergehenden Ansprüche, wobei der erste Kanal (7) und der zweite Kanal (8) sich in die Kammer (4) unterschiedlich weit hineinragend erstrecken, unterschiedlich ausgerichtet sind und/oder sich aus unterschiedlichen Seitenwänden in die Kammer (4) hineinragend erstrecken.

8. Beatmungsgerät (1) nach einem der vorhergehenden Ansprüche, wobei sich der erste Kanal (7) und/oder der zweite Kanal (8) außerhalb der Kammer (4) aus einer Wandung der Kammer (4) heraus erstreckt.

9. Beatmungsgerät (1) nach einem der vorhergehenden Ansprüche, wobei der erste Kanal (7) und/oder der zweite Kanal (8) einen gekrümmten oder einen gewellten Verlauf aufweist.

10. Beatmungsgerät (1) nach einem der vorhergehenden Ansprüche, wobei der erste Kanal (7) und/oder der zweite Kanal (8) eine Querschnittsfläche orthogonal zu einer Längsrichtung des ersten Kanals (7) bzw. des zweiten Kanals (8) aufweist, wobei die Querschnittsfläche zumindest abschnittsweise eine Form aufweist, die ausgewählt ist aus der Gruppe: rechteckig, schlitzförmig, sichelförmig, oval, Kreuzförmig, X-förmig, Stern-förmig, runder oder eckiger Ringspalt, L-förmig, Hantel-förmig, U-förmig, V-förmig, T-förmig, Ellipsen-förmig, Trapez-förmig.

11. Beatmungsgerät (1) nach Anspruch 10, wobei die Querschnittsformen des ersten Kanals (7) und des zweiten Kanals (8) identisch oder unterschiedlich ausgebildet sind.

12. Beatmungsgerät (1) nach einem der vorhergehenden Ansprüche, wobei der erste Kanal (7) und/oder der zweite Kanal (8) ein Volumen aufweisen, wobei das Volumen eine Tiefe von 1 mm - 50 mm, bevorzugt 1 mm - 20 mm, besonders bevorzugt 3 mm - 7 mm, eine Breite von 5 mm - 100 mm, bevorzugt 20 mm - 60 mm, und eine Länge von 1 mm - 100 mm, bevorzugt 20 mm - 60 mm, aufweist.

13. Beatmungsgerät (1) nach einem der vorhergehenden Ansprüche, wobei zumindest ein Kanal eine Querschnittsfläche (A) und einen Umfang (P) aufweist, die durch das Verhältnis (R), des Umfangs (P) im Quadrat, zur Querschnittsfläche (A) charakterisiert werden entsprechend der Formel $R = P^2/A$ wobei dieses Verhältnis (R) einen Wert von über 14, bevorzugt 16 oder über 16, besonders bevorzugt 20 oder über 20, stärker bevorzugt 30 oder über 30 einnimmt.

## Claims

1. A respiratory apparatus (1) comprising an inlet (6), an outlet (3), and a blower (5), wherein a breathing gas path is formed between the inlet (6) and the outlet (3) and is conducted through the blower (5), wherein along the breathing gas path there are disposed a chamber (4), a first channel (7) opening into the chamber (4), and a second channel (8) opening into the chamber (4), and wherein a first acoustic impedance of the first channel (7) and a second acoustic impedance of the second channel (8) differ from a third acoustic impedance of the chamber (4) such that sound generated by the blower (5) is attenuated, wherein the blower (5) is disposed in the chamber (4), wherein the first channel (7) and the second channel (8) extend so as to project into the chamber (4), wherein a first opening (13) of the first channel (7) and a second opening (14) of the second channel (8) are disposed such that, during operation of the respiratory apparatus (1), a breathing gas flow is established that at least in sections flows around the blower (5), wherein the breathing gas path between the second channel (8) and the outlet (3) is conducted through the blower (5).

2. The respiratory apparatus (1) according to claim 1, wherein a blower body (18) of the blower (5) is mounted in the chamber (4) in a vibration-attenuating manner.

3. The respiratory apparatus (1) according to one of the preceding claims, wherein the chamber (4) has a volume of 100 cm$^3$ - 5000 cm$^3$, preferably 500 cm$^3$ - 3000 cm$^3$, particularly preferably 500 - 1200 cm$^3$.

4. The respiratory apparatus (1) according to one of the preceding claims, wherein the breathing gas path between the inlet (6) and the chamber (4) is formed at least in sections by the first channel (7).

5. The respiratory apparatus (1) according to one of the preceding claims, wherein the breathing gas path between the outlet (3) and the chamber (4) is formed at least in sections by the second channel (8).

6. The respiratory apparatus (1) according to one of the preceding claims, wherein the second channel (8) opens into an auxiliary chamber (9), wherein a fourth acoustic impedance of the auxiliary chamber (9) differs from the second acoustic impedance of the second channel (8) such that the sound generated by the blower (5) is additionally attenuated, wherein the auxiliary chamber (9) has a volume of 10 cm$^3$ to 200 cm$^3$, preferably 30 cm$^3$ to 100 cm$^3$.

7. The respiratory apparatus (1) according to one of the preceding claims, wherein the first channel (7) and the second channel (8) extend so as to project into the chamber (4) to different extents, are oriented differently, and/or extend into the chamber (4) from different side walls.

8. The respiratory apparatus (1) according to one of the preceding claims, wherein the first channel (7) and/or the second channel (8) extends from a wall of the chamber (4) to the outside of the chamber (4).

9. The respiratory apparatus (1) according to one of the preceding claims, wherein the first channel (7) and/or the second channel (8) has a curved or waved profile.

10. The respiratory apparatus (1) according to one of the preceding claims, wherein the first channel (7) and/or the second channel (8) has a cross-sectional area that is orthogonal to a longitudinal direction of the first channel (7) or the second channel (8), respectively, wherein the cross-sectional area has, at least in sections, a shape selected from the group consisting of: rectangular, slot-shaped, crescent-shaped, oval, cross-shaped, X-shaped, star-shaped, round or angular annular gap, L-shaped, dumbbell-shaped, U-shaped, V-shaped, T-shaped, elliptical, trapezoidal.

11. The respiratory apparatus (1) according to claim 10, wherein the cross-sectional shapes of the first channel (7) and of the second channel (8) are designed to be identical or different.

12. The respiratory apparatus (1) according to one of the preceding claims, wherein the first channel (7) and/or the second channel (8) has a volume, wherein the volume has a depth of 1 mm - 50 mm, preferably 1 mm - 20 mm, more preferably 3 mm - 7 mm, a width of 5 mm - 100 mm, preferably 20 mm - 60 mm, and a length of 1 mm - 100 mm, preferably 20 mm - 60 mm.

**13.** The respiratory apparatus (1) according to one of the preceding claims, wherein at least one channel has a cross-sectional area (A) and a perimeter (P) which are **characterized by** the ratio (R) of the square of the perimeter (P) to the cross-sectional area (A), according to the formula $R = P^2/A$ wherein this ratio (R) has a value greater than 14, preferably 16 or greater than 16, more preferably 20 or greater than 20, even more preferably 30 or greater than 30.

## Revendications

**1.** Appareil respiratoire (1) comprenant un orifice d'admission (6), un orifice de sortie (3) et un ventilateur (5), dans lequel un trajet de gaz respiratoire est formé entre l'orifice d'admission (6) et l'orifice de sortie (3), lequel est guidé à travers le ventilateur (5), dans lequel une chambre (4), un premier canal (7) débouchant dans la chambre (4) et un deuxième canal (8) débouchant dans la chambre (4) sont disposés le long du trajet de gaz respiratoire, et dans lequel une première impédance acoustique du premier canal (7) et une deuxième impédance acoustique du deuxième canal (8) diffèrent d'une troisième impédance acoustique de la chambre (4), de telle sorte qu'un bruit généré par le ventilateur (5) est atténué, dans lequel le ventilateur (5) est disposé dans la chambre (4), dans lequel le premier canal (7) et le deuxième canal (8) s'étendent de manière à faire saillie dans la chambre (4), dans lequel une première ouverture (13) du premier canal (7) et une deuxième ouverture (14) du deuxième canal (8) sont disposées de telle sorte que lors du fonctionnement de l'appareil respiratoire (1), il s'établit un flux de gaz respiratoire s'écoulant au moins partiellement autour du ventilateur (5), dans lequel le trajet de gaz respiratoire est guidé entre le deuxième canal (8) et l'orifice de sortie (3) à travers le ventilateur (5).

**2.** Appareil respiratoire (1) selon la revendication 1, dans lequel un corps de ventilateur (18) du ventilateur (5) est fixé dans la chambre (4) de manière à amortir les vibrations.

**3.** Appareil respiratoire (1) selon l'une des revendications précédentes, dans lequel la chambre (4) présente un volume de 100 cm$^3$ à 5000 cm$^3$, de préférence de 500 cm$^3$ à 3000 cm$^3$, de façon particulièrement préférée de 500 à 1200 cm$^3$.

**4.** Appareil respiratoire (1) selon l'une des revendications précédentes, dans lequel le trajet de gaz respiratoire entre l'orifice d'admission (6) et la chambre (4) est formé, au moins par sections, par le premier canal (7).

**5.** Appareil respiratoire (1) selon l'une des revendications précédentes, dans lequel le trajet de gaz respiratoire entre l'orifice de sortie (3) et la chambre (4) est formé, au moins par sections, par le deuxième canal (8).

**6.** Appareil respiratoire (1) selon l'une des revendications précédentes, dans lequel le deuxième canal (8) débouche dans une chambre additionnelle (9), dans lequel une quatrième impédance acoustique de ladite chambre additionnelle (9) diffère de la deuxième impédance acoustique du deuxième canal (8), de sorte que le bruit généré par le ventilateur (5) est en outre atténué, dans lequel ladite chambre additionnelle (9) présente un volume de 10 cm$^3$ à 200 cm$^3$, de préférence de 30 cm$^3$ à 100 cm$^3$.

**7.** Appareil respiratoire (1) selon l'une des revendications précédentes, dans lequel le premier canal (7) et le deuxième canal (8) s'étendent de manière à faire saillie à des distances différentes dans la chambre (4), sont orientés différemment et/ou s'étendent de manière à faire saillie à partir de parois latérales différentes dans la chambre (4).

**8.** Appareil respiratoire (1) selon l'une des revendications précédentes, dans lequel le premier canal (7) et/ou le deuxième canal (8) s'étendent à l'extérieur de la chambre (4) à partir d'une paroi de la chambre (4).

**9.** Appareil respiratoire (1) selon l'une des revendications précédentes, dans lequel le premier canal (7) et/ou le deuxième canal (8) présentent un tracé courbe ou ondulé.

**10.** Appareil respiratoire (1) selon l'une des revendications précédentes, dans lequel le premier canal (7) et/ou le deuxième canal (8) présentent une surface de section transversale orthogonale à une direction longitudinale du premier canal (7) ou, respectivement, du deuxième canal (8), dans lequel la surface de section transversale présente au moins par sections une forme choisie à partir du groupe suivant : rectangulaire, en fente, en croissant, ovale, en croix, en X, en étoile, en fente annulaire circulaire ou angulaire, en L, en forme d'haltère, en U, en V, en T, elliptique, trapézoïdale.

**11.** Appareil respiratoire (1) selon la revendication 10, dans lequel les formes de section transversale du premier canal (7) et du deuxième canal (8) sont réalisées de manière identique ou différente.

**12.** Appareil respiratoire (1) selon l'une des revendications précédentes, dans lequel le premier canal (7) et/ou le deuxième canal (8) présentent un volume, dans lequel le volume présente une profondeur de 1 mm à 50 mm, de préférence de 1 mm à 20 mm, de façon particulièrement préférée de 3 mm à 7 mm, une largeur de 5 mm à 100 mm, de préférence de 20 mm à 60 mm, et une longueur de 1 mm à 100 mm, de préférence de 20 mm à 60 mm.

13. Appareil respiratoire (1) selon l'une des revendications précédentes, dans lequel au moins un canal présente une surface de section transversale (A) et un périmètre (P) **caractérisés par** le rapport (R) du périmètre (P) au carré à la surface de section transversale (A) selon la formule $R = P^2/A$ dans lequel ce rapport (R) prend une valeur supérieure à 14, de préférence de 16 ou supérieure à 16, de façon particulièrement préférée de 20 ou supérieure à 20, de façon encore plus préférée de 30 ou supérieure à 30.

Fig. 1

Fig. 2

Fig. 3

Fig. 4

Fig. 5

Fig. 6

Fig. 7

Fig. 8

Fig. 9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1457222 B1
- US 2016015919 A1
- US 2014299132 A1
- US 2004035422 A1
- US 2015023782 A1